# EUROPEAN PATENT SPECIFICATION

(11) **EP 3 443 356 B1**
(45) Date of publication and mention of the grant of the patent: **05.06.2024**
(21) Application number: 17782034.7
(22) Date of filing: 13.04.2017
(51) Int. Cl.: G01N 33/68

(54) **HOST BIOMARKERS FOR IMMUNODIAGNOSIS AND MONITORING OF TUBERCULOSIS DISEASE**
HOST-BIOMARKER ZUR IMMUNDIAGNOSE UND ÜBERWACHUNG DER TUBERKULOSEERKRANKUNG
BIOMARQUEURS HÔTES POUR L'IMMUNODIAGNOSTIC ET LA SURVEILLANCE DE LA TUBERCULOSE

(30) Priority: 15.04.2016 ZA 201602557
(43) Date of publication of application: 20.02.2019
(73) Proprietor: Stellenbosch University, 7600 Western Cape Province (ZA)
(72) Inventor: JACOBS, Ruschca, 7600 Stellenbosch (ZA); WALZL, Gerhard, 7550 Durbanville (ZA); CHEGOU, Novel Njweipi, 7530 Bellville (ZA)
(74) Representative: Gill Jennings & Every LLP
(86) International application number: PCT/IB2017/052142
(87) International publication number: WO 2017/179008

(56) References cited:
- WO-A1-2013/155460
- WO-A1-2014/020343
- WO-A1-2014/133855
- WO-A1-2015/128830
- WO-A1-2015/128830
- WO-A1-2016/162827
- WO-A2-2013/175459
- FIGEN DEVECI ET AL: "Lymphocyte Subpopulations in Pulmonary Tuberculosis Patients", MEDIATORS OF INFLAMMATION., vol. 2006, 1 January 2006 (2006-01-01), pages 1-6, XP055433790, GB ISSN: 0962-9351, DOI: 10.1155/MI/2006/89070
- H. VEENSTRA ET AL: "Changes in leucocyte and lymphocyte subsets during tuberculosis treatment; prominence of CD3dimCD56+ natural killer T cells in fast treatment responders", CLINICAL AND EXPERIMENTAL IMMUNOLOGY, vol. 145, no. 2, 1 August 2006 (2006-08-01), pages 252-260, XP055433796, GB ISSN: 0009-9104, DOI: 10.1111/j.1365-2249.2006.03144.x
- Anonymous: "Human N-CAM1 ELISA Kit Product EHNCAM1 - Data sheet- from Thermo Scientific", , 9 April 2015 (2015-04-09), pages 1-8, XP055639463, Retrieved from the Internet: URL:https://assets.thermofisher.com/TFS-As sets/LSG/manuals/EHNCAM1.pdf [retrieved on 2019-11-06]
- CORSTJENS PAUL L A M ET AL: "Multi-center evaluation of a user-friendly lateral flow assay to determine IP-10 and CCL4 levels in blood of TB and non-TB cases in Africa", CLINICAL BIOCHEMISTRY, ELSEVIER INC, US, CA, vol. 49, no. 1, 15 August 2015 (2015-08-15), pages 22-31, XP029381098, ISSN: 0009-9120, DOI: 10.1016/J.CLINBIOCHEM.2015.08.013
- RUSCHCA JACOBS ET AL: "Identification of novel host biomarkers in plasma as candidates for the immunodiagnosis of tuberculosis disease and monitoring of tuberculosis treatment response", ONCOTARGET, vol. 7, no. 36, 6 September 2016 (2016-09-06), pages 57581-57592, XP055433820, DOI: 10.18632/oncotarget.11420
- DEVECI, F. ET AL.: 'Lymphocyte Subpopulations in Pulmonary Tuberculosis Patients' MEDIATORS OF INFLAMMATION vol. 2006, 2006, pages 1 - 6, XP055433790
- VEENSTRA, H. ET AL.: 'Changes in leucocyte and lymphocyte subsets during tuberculosis treatment; prominence of CD 3dim CD 56+ natural killer T cells in fast treatment responders' CLINICAL AND EXPERIMENTAL IMMUNOLOGY vol. 145, no. 2, 2006, pages 252 - 260, XP055433796
- MENDEZ, A. ET AL.: 'CCL2, CCL18 and sIL4R in renal, meningeal and pulmonary TB; a 2 year study of patients and contacts' TUBERCULOSIS vol. 91, no. 2, 28 December 2011, pages 140 - 145, XP028175614
- NGUYEN HOANG, A.T. ET AL.: 'PA11/41 Lung tissue stromal cells affect CCL18 production by dendritic cells in steady state and response to mycobacterium tuberculosis' EUROPEAN JOURNAL OF IMMUNOLOGY vol. 39, no. SUPPL, 2009, pages S332 - S555, XP055433810
- JACOBS, R. ET AL.: 'Identification of novel host biomarkers in plasma as candidates for the immunodiagnosis of tuberculosis disease and monitoring of tuberculosis treatment response' ONCOTARGET vol. 7, no. 36, August 2016, pages 57581 - 57592, XP055433820
- JACOBS, R. ET AL.: 'Diagnostic potential of novel salivary host biomarkers as candidates for the immunological diagnosis of tuberculosis disease and monitoring of tuberculosis treatment response' PLOS ONE vol. 11, no. 8, August 2016, XP055433822
- Gerhard Walzl ET AL: "Immunological biomarkers of tuberculosis", Nature Reviews Immunology, vol. 11, no. 5, 8 April 2011 (2011-04-08), pages 343-354, XP055109445, ISSN: 1474-1733, DOI: 10.1038/nri2960
- JAYNE S. SUTHERLAND ET AL: "Highly Accurate Diagnosis of Pleural Tuberculosis by Immunological Analysis of the Pleural Effusion", PLOS ONE, vol. 7, no. 1, 25 January 2012 (2012-01-25), page e30324, XP055154918, DOI: 10.1371/journal.pone.0030324

## Description

### FIELD OF THE INVENTION

The invention relates to host biomarkers which can be used for diagnosing tuberculosis (TB) disease or monitoring the response to TB treatment.

### BACKGROUND OF THE INVENTION

Tuberculosis (TB) disease, although curable, still accounted for the deaths of 1 .5 million people in 2014 [1]. Rapid and accurate tools are urgently needed for early diagnosis of the disease and monitoring of the response to treatment.

The gold standard test for TB (culture) is not widely available, especially in resource-poor settings. The Ziehl Nielsen sputum smear test is often the only available diagnostic tool in these settings, even though its limitations are well publicised [2]. Month 2 culture conversion is the most investigated biomarker for TB treatment response, but the wide unavailability of culture and its long turn-around time are serious limitations. Smear microscopy is not very useful for monitoring anti-TB treatment response as it is unable to distinguish live from dead bacilli [3]. The development of the XpertMTB/RI F test (Cepheid Inc., Sunnyvale, USA) was a significant advance in the TB diagnostic field, as the test yields results within 2 hours, coupled with the detection of resistance to rifampicin, as a proxy for multi-drug resistant TB [4]. However, the high operating costs and need for infrastructure are major obstacles for its implementation in resource-poor settings. The Xpert test is also not useful in monitoring of TB treatment response, as the test cannot distinguish between DNA from dead and live bacteria [5]. Immunodiagnostic techniques may be useful in both the diagnosis of TB disease and monitoring of the response to treatment, especially as they may be easily adaptable into rapid, point-of-care tests, which would be suitable in resource-constrained settings. Furthermore, such tests will also be beneficial in cases where a conventional sputum-based diagnosis (smear microscopy, culture or Xpert) is difficult, e.g. in paediatric TB and in individuals with extrapulmonary TB.

IFN-gamma (IFN-y) release assays (IGRA) remain the most widely used commercial immunodiagnostic tests for TB. These assays have been shown to be useful in the diagnosis of infection with Mycobacterium tuberculosis (MTB) but as they cannot discriminate between active TB disease and latent MTB infection, they are of limited value in high TB-endemic areas. The use of IGRAs as tools for monitoring of the response to TB treatment has so far yielded conflicting results [6][7]. An important limitation of overnight culture-based assays such as IGRAs is the fact that they are not suitable as point-of-care tests. These tests are therefore not ideal for resource-constrained settings. The potential value of diagnostic approaches that are based on the detection of host biomarkers ex vivo, in easily obtainable samples such as saliva, serum or plasma has been demonstrated in previous studies [8][9][10]. Such host biomarker-based tests in addition to being used as diagnostic tests for TB, may also be useful in monitoring of the response to TB treatment [8][11][12]. Despite the promise shown so far in these previous investigations, no validated diagnostic tests based on the detection of host biomarkers in unstimulated samples currently exist. WO2015/128830 A1 discloses methods of diagnosing TB using various panels of biomarkers in unstimulated blood samples from subjects.

There is therefore still a need to identify new candidate host markers that might be useful in the diagnosis of TB disease.

### SUMMARY OF THE INVENTION

According to a first embodiment of the invention, there is provided a method of diagnosing tuberculosis disease (TB) in a subject, the method comprising the steps of:
a) testing a blood sample from a subject suspected of having TB for the presence of neural cell adhesion molecule (NCAM/CD56); and
b) diagnosing the patient as having TB if a reduced level of NCAM is detected in the sample relative to a level of NCAM which is associated with subjects with upper and lower respiratory tract infections, chronic obstructive pulmonary disease and asthma.

The sample may be tested for the presence of NCAM and at least two other biomarkers, at least three other biomarkers, at least four other biomarkers, at least five other biomarkers, or at least six other biomarkers, the other biomarkers being selected from ferritin, SAP, A2M, IL-22, CFH, ECM-1 , IL-1 β, sCD40L,IL-13, Apo-A-1 , myoglobin, IL-12(p40) and MIP-4/CCL18.

For example, the sample may be tested for the presence of NCAM and:
at least two other biomarkers selected from ferritin, SAP, A2M, IL-22, CFH, ECM-1, IL-1β, sCD40L, IL-13, Apo-A-1, myoglobin, IL-12(p40), MIP-1β, CRP, PCT, TPA, SAA, ADAMTS-13, p-selectin, GDF-15, I-309, IFN-γ, IP-10, TNF-α, MIG, ITAC, HCC-1, antithrombin III, transthyretin, BDNF, sFas, lipocalin-2, VEGF, PEDF, CC4, IL-33, TNF-β and SDF-1 α;
at least three other biomarkers selected from ferritin, SAP, A2M, IL-22, CFH, ECM-1, IL-1 β, sCD40L, IL-13, Apo-A-1, myoglobin, IL-12(p40), MIP-4/CCL18, CRP, PCT, TPA, SAA, ADAMTS-13, p-selectin, GDF-15, I-309, IFN-γ, IP-10, TNF-α, MIG, ITAC, HCC-1, antithrombin III, transthyretin, BDNF, sFas, lipocalin-2, VEGF, PEDF, CC4, IL-33, TNF-β, MIP-1β and SDF-1 α;
at least four other biomarkers selected from ferritin, SAP, A2M, IL-22, CFH, ECM-1, IL-1 β, sCD40L, IL-13, Apo-A-1, myoglobin, IL-12(p40), MIP-4/CCL18, CRP, PCT, TPA, SAA, ADAMTS-13, p-selectin, GDF-15, I-309, IFN-γ, IP-10, TNF-α, MIG, ITAC, HCC-1, antithrombin III, transthyretin, BDNF, sFas, lipocalin-2, VEGF, PEDF, CC4, IL-33, TNF-β, MIP-1β and SDF-1 α; or
at least five other biomarkers selected from ferritin, SAP, A2M, IL-22, CFH, ECM-1, IL-1 β, sCD40L, IL-13, Apo-A-1, myoglobin, IL-12(p40), MIP-4/CCL18, CRP, PCT, TPA, SAA, ADAMTS-13, p-selectin, GDF-15, I-309, IFN-γ, IP-10, TNF-α, MIG, ITAC, HCC-1, antithrombin III, transthyretin, BDNF, sFas, lipocalin-2, VEGF, PEDF, CC4, IL-33, TNF-β, MIP-1β and SDF-1 α.

It is stated herein that another term for MIP-4 may be CCL18.

At least one of the other biomarkers may be ferritin or SAP.

For example, the sample may be tested for the following biomarkers:
NCAM, SAP, ferritin, CFH and ECM-1;
NCAM, SAP, IL-1 β, sCD40L, IL-13 and Apo A-1;
NCAM, A2M, IL-22, ferritin, myoglobin and IL-12(p40); or
NCAM, A2M, IL-22, ferritin, TNF-β and MIP-4/CCL18.

The method of diagnosing tuberculosis disease (TB) in a subject may further comprise the steps of:
a) in addition to testing said blood sample from a subject suspected of having TB for the presence of NCAM, testing a blood sample for at least one other biomarker selected from ferritin, serum amyloid P (SAP), alpha-2-macroglobulin (A2M), interleukin-22 (IL-22), complement factor H (CFH), extracellular matrix protein 1 (ECM-1), interleukin-1 β (IL-1 β), soluble CD40 ligand (sCD40L), interleukin-13 (IL-13), apolipoprotein A-1 (Apo-A-1), myoglobin, interleukin-12 (IL-12(p40)), macrophage inflammatory protein-4 (MIP-4/CCL18), antithrombin III, growth differentiation factor (GDF-15), hemofiltrate CC chemokine-1 (HCC1), C-reactive protein (CRP), procalcitonin (PCT), tissue plasminogen activator (TPA), serum amyloid A (SAA), a disintegrin and metalloproteinase with a thrombospondin type 1 motif, member 13 (ADAMTS-13 / von Willebrand factor-cleaving protease (VWFCP)), p-selectin, Chemokine (C-C motif) ligand 1 (CCL1 / 1-309), interferon γ (IFN-γ), interferon gamma inducible protein 10 (IP-10), tumor necrosis factor α (TNF-α), monokine induced by gamma interferon (MIG), interferon inducible T-cell alpha chemoattractant (ITAC), transthyretin, brain-derived neurotrophic factor (BDNF), soluble Fas (sFas), lipocalin-2, vascular endothelial growth factor (VEGF), pigment epithelium derived factor (PEDF), complement component 4 (CC4), interleukin-33 (IL-33), tumor necrosis factor β (TNF-β), macrophage inflammatory protein 1β (MIP-1β) and stromal cell derived factor-1 alpha (SDF-1 α); and
b) diagnosing the patient as having TB if NCAM at a said reduced level and at least one of the biomarkers is detected in said sample.

The patient may be diagnosed as having TB if at least two, at least three, at least four, at least five or at least six of the biomarkers are detected in the sample.

The sample may be a whole blood, plasma or serum sample, and is preferably an unstimulated sample.

The TB may be pulmonary TB.

The method may distinguish TB from latent Mycobacterium tuberculosis infection.

A capture agent specific for each of the biomarkers may be used to bind each of the respective biomarkers. One or more indicators may be provided to indicate when binding of each of the capture agents and biomarkers occurs.

According to a reference example which is not included in the scope of the present invention, there is provided a device for diagnosing tuberculosis disease (TB) according to the methods described above, the device comprising:
a) a means for receiving a blood sample from a subject;
b) capture agent(s) for binding at least NCAM and optionally other biomarkers selected from ferritin, SAP, A2M, IL-22, CFH, ECM-1 , IL-1 β, sCD40L,IL-13, Apo-A-1 , myoglobin, IL-12(p40) and MIP-4/CCL18; and
c) at least one indicator which indicates when the capture agents bind to the biomarkers.

The device may include measuring means for measuring the levels of the detected biomarkers.

The device may further include amplifying means for increasing the sensitivity of the detection of the biomarkers.

According to a further embodiment of the invention, there is provided a use of a kit for diagnosing tuberculosis disease (TB) in a blood sample from a subject according to the methods described above, the kit comprising:
a) a means of receiving a sample from a patient;
b) a capture agent for binding NCAM and optionally also capture agents for binding other biomarkers selected from ferritin, SAP, A2M, IL-22, CFH, ECM-1 , IL-1 β, sCD40L,IL-13, Apo-A-1 , myoglobin, IL-12(p40) and MIP-4/CCL18; and
c) at least one indicator which indicates when the capture agents bind to the biomarkers.

The kit may also include:
a) means for obtaining the blood sample from the patient,
b) a blood sample collection device,
c) a buffer for mixing with the blood sample, and
d) a membrane/test strip with antibodies for the biomarker(s).

Use of the kit may also include the use of a device for performing the method and/or instructions for performing the method of diagnosis.

### BRIEF DESCRIPTION OF THE FIGURES

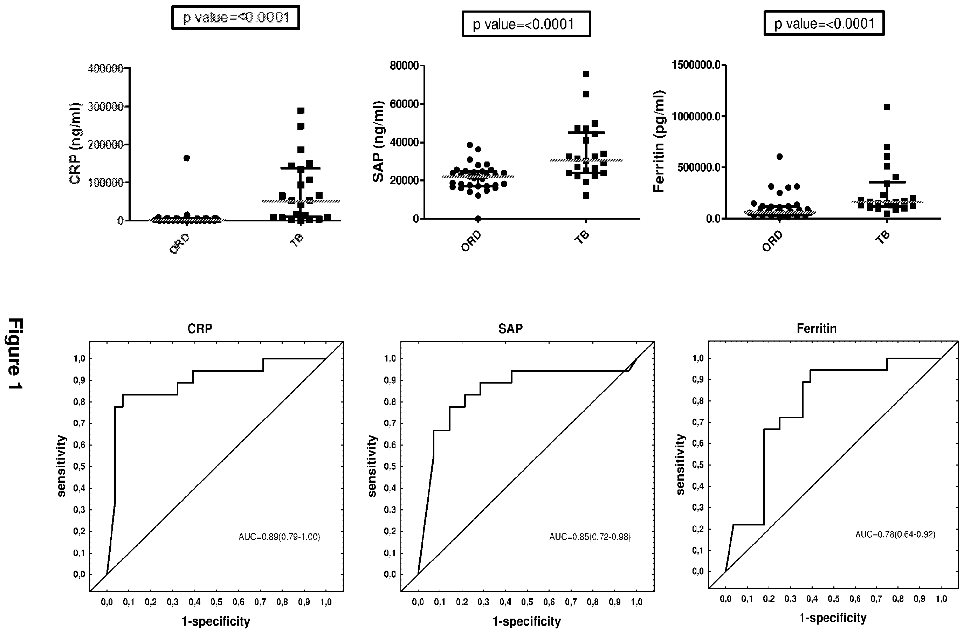

**Figure 1****:** shows concentrations of host markers detected in plasma samples from TB patients (n=22) and individuals with other respiratory diseases (n=33) and receiver operator characteristics curves showing the accuracies of these markers in the diagnosis of TB disease. Representative plots are shown for CRP, SAP and ferritin. Error bars in the scatter dot plots represent the median with interquartile range.

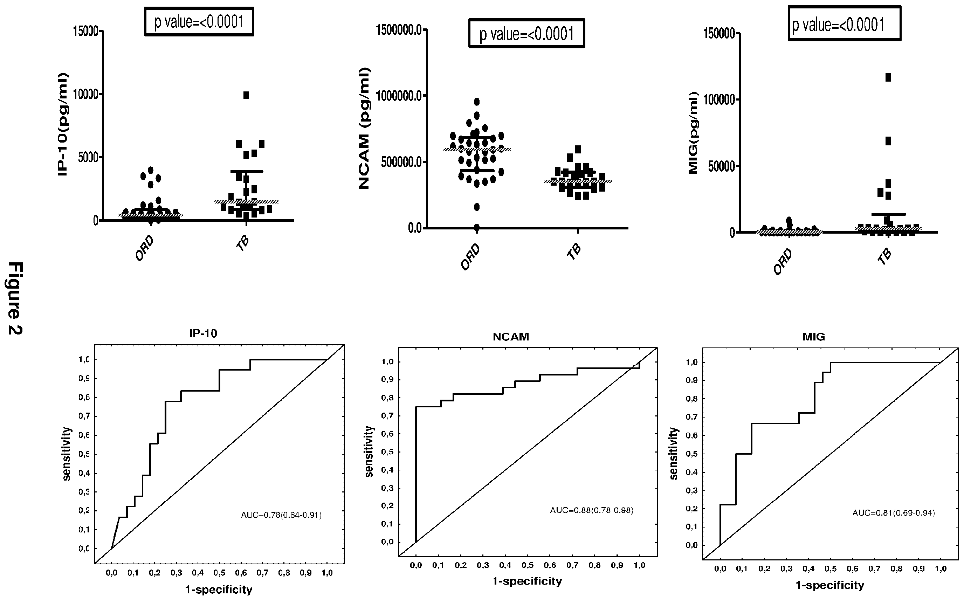

**Figure 2****:** shows concentrations of host markers detected in plasma samples from TB patients (n=22) and individuals with other respiratory diseases (n=33) and receiver operator characteristics curves showing the accuracies of these markers in the diagnosis of TB disease. Representative plots are shown for IP-10, NCAM and MIG. Error bars in the scatter dot plots represent the median with interquartile range.

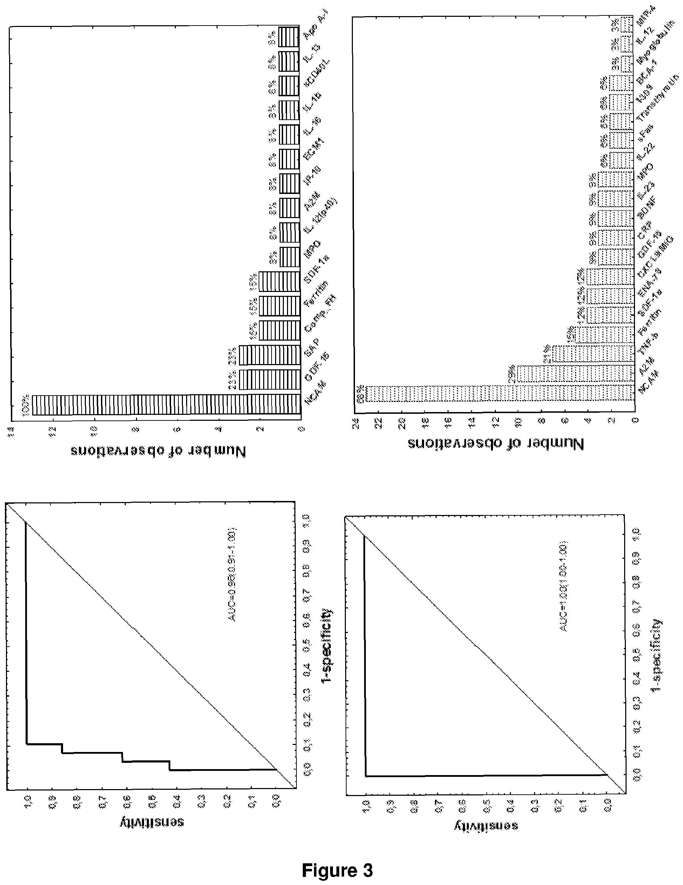

**Figure 3****:** shows the accuracy of multi-marker models in the diagnosis of TB disease. A receiver operator characteristics (ROC) curve shows the accuracy of the most accurate six-marker biosignature (NCAM, SAP, IL-1 β, sCD40L, IL-13 and Apo A-1 ) in the diagnosis of TB disease in all study participants, regardless of HIV infection status (A), frequency of analytes in the top 13 general discriminant analysis (GDA) models that most accurately classified study participants as TB disease or ORD irrespective of HIV status (B), ROC curve showing the accuracy of the most accurate six-marker biosignature (NCAM+A2M+IL-22+ferritin+ myoglobin+IL-12(p40) or NCAM+A2M+IL-22+ferritin+TNF- +MIP-4/CCL18) in the diagnosis of TB disease in HIV negative study participants (C), and frequency of analytes in the top 34 GDA models that most accurately classified study participants as TB disease or ORD in the absence of HIV infection (D). The bar graphs (B and D) indicate the frequency of analytes in the most accurate GDA models.

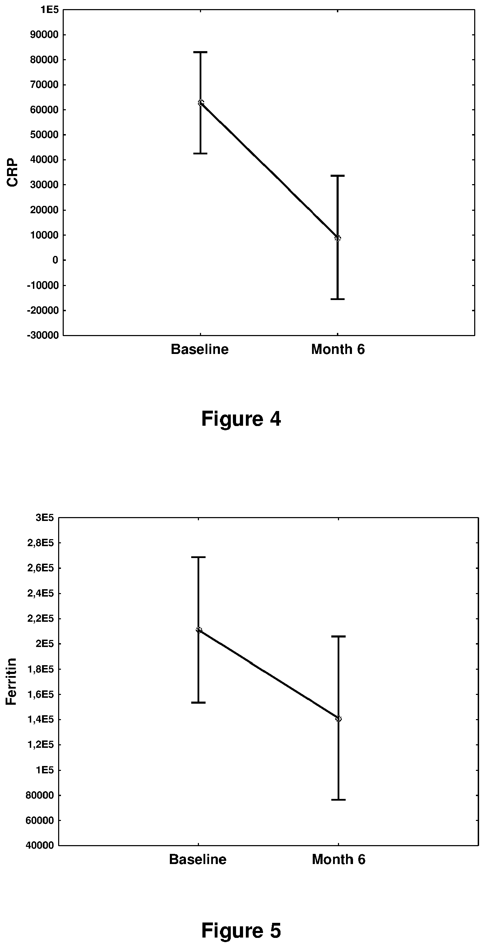

**Figure 4****:** shows before (baseline) and after treatment (month 6) concentrations of CRP in plasma samples from TB patients. Plasma was collected from patients at recruitment, prior to the initiation of anti-TB therapy and then at the end of standard TB treatment (month 6). Error bars indicate the Least Squared means with 95% Confidence Intervals.
**Figure 5****:** shows before (baseline) and after treatment (month 6) concentrations of ferritin in plasma samples from TB patients. Plasma was collected from patients at recruitment, prior to the initiation of anti-TB therapy and then at the end of standard TB treatment (month 6). Error bars indicate the Least Squared means with 95% Confidence Intervals.

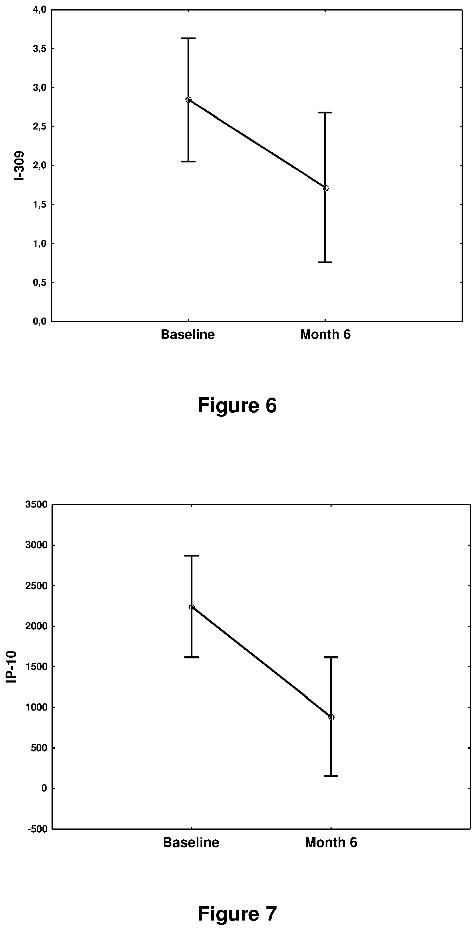

**Figure 6****:** shows before (baseline) and after treatment (month 6) concentrations of I-309 in plasma samples from TB patients. Plasma was collected from patients at recruitment, prior to the initiation of anti-TB therapy and then at the end of standard TB treatment (month 6). Error bars indicate the Least Squared means with 95% Confidence Intervals.
**Figure 7****:** shows before (baseline) and after treatment (month 6) concentrations of IP-10 in plasma samples from TB patients. Plasma was collected from patients at recruitment, prior to the initiation of anti-TB therapy and then at the end of standard TB treatment (month 6). Error bars indicate the Least Squared means with 95% Confidence Intervals.

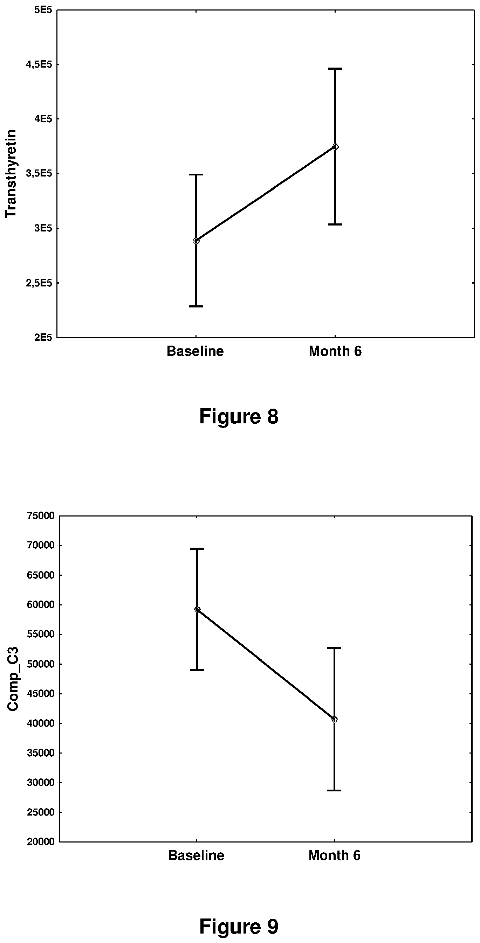

**Figure 8****:** shows before (baseline) and after treatment (month 6) concentrations of transthyretin in plasma samples from TB patients. Plasma was collected from patients at recruitment, prior to the initiation of anti-TB therapy and then at the end of standard TB treatment (month 6). Error bars indicate the Least Squared means with 95% Confidence Intervals.
**Figure 9****:** shows before (baseline) and after treatment (month 6) concentrations of Comp_C3 in plasma samples from TB patients. Plasma was collected from patients at recruitment, prior to the initiation of anti-TB therapy and then at the end of standard TB treatment (month 6). Error bars indicate the Least Squared means with 95% Confidence Intervals.

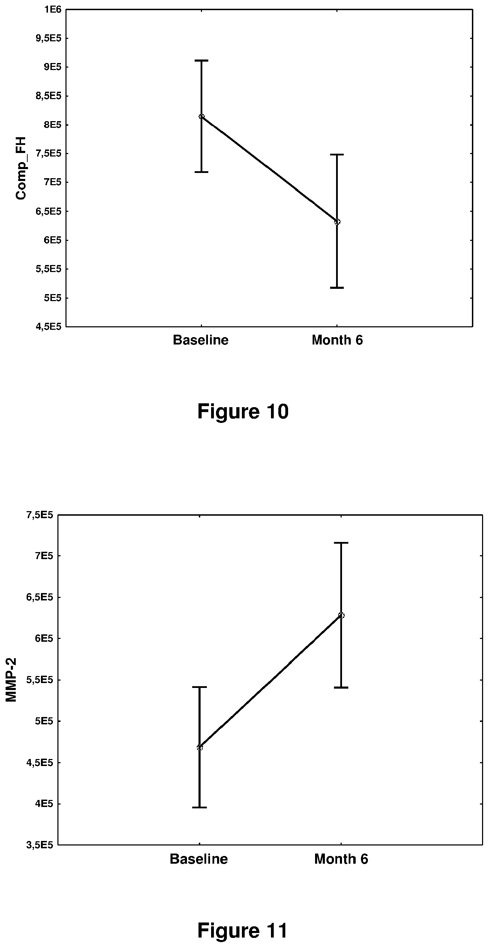

**Figure 10****:** shows before (baseline) and after treatment (month 6) concentrations of Comp-FH in plasma samples from TB patients. Plasma was collected from patients at recruitment, prior to the initiation of anti-TB therapy and then at the end of standard TB treatment (month 6). Error bars indicate the Least Squared means with 95% Confidence Intervals.
**Figure 11****:** shows before (baseline) and after treatment (month 6) concentrations of MMP-2 in plasma samples from TB patients. Plasma was collected from patients at recruitment, prior to the initiation of anti-TB therapy and then at the end of standard TB treatment (month 6). Error bars indicate the Least Squared means with 95% Confidence Intervals.

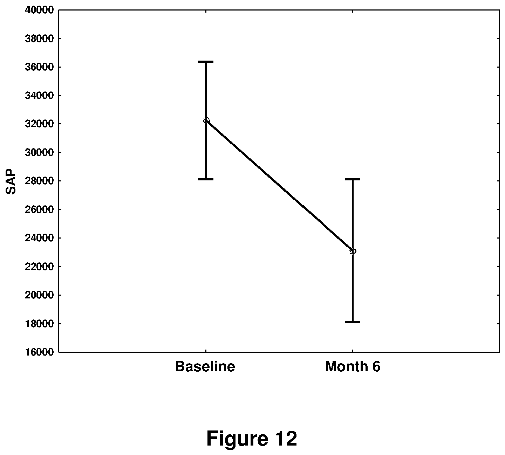

**Figure 12****:** shows before (baseline) and after treatment (month 6) concentrations of SAP in plasma samples from TB patients. Plasma was collected from patients at recruitment, prior to the initiation of anti-TB therapy and then at the end of standard TB treatment (month 6). Error bars indicate the Least Squared means with 95% Confidence Intervals.

### DETAILED DESCRIPTION OF THE INVENTION

Neural cell adhesion molecule CD56 (NCAM) is described herein as a biomarker for diagnosing tuberculosis disease (TB) in a subject. The method of diagnosis detects NCAM in a single biomarker test or as one biomarker in a panel of 2, 3, 4, 5, 6 or even more biomarkers. The method of diagnosis can be used to distinguish active TB disease from latent Mycobacterium tuberculosis infection in the subject.

Throughout the specification and claims unless the contents requires otherwise the word 'comprise' or variations such as 'comprises' or 'comprising' will be understood to imply the inclusion of a stated integer or group of integers but not the exclusion of any other integer or group of integers.

When the diagnostic test detects NCAM as one of a panel of biomarkers, the other biomarkers can be selected from: CRP, SAP, PCT, ferritin, TPA, SAA, ADAMTS-13, p-selectin, GDF-15, I-309, IFN-Y, IP-10, TNF-a, CFH, MIG, ITAC, HCC-1 , MIP-4/CCL18, antithrombin III, Apo A-1 , transthyretin, BDNF, sFas, lipocalin-2, VEGF, PEDF, CC4, IL-33, A2M, IL-22, ECM-1 , IL-1 β, SCD40L, IL-13, myoglobin, IL-12(p40), TNF-β, MIP-1 β, VEGF and SDF-1 a.

In one embodiment, the other biomarkers are selected from CRP, SAP, PCT, ferritin, TPA, SAA, I-309, MIG, MIP-4/CCL18, Apo A-1 , transthyretin, CFH, TNF-a, IP-10, antithrombin III, GDF-15, HCC1 , ECM-1 , II_-1 β, sCD40L and IL-13.

In one embodiment, the other biomarkers are selected from CRP, SAP, PCT, ferritin, TPA, SAA, I-309, MIG, MIP-4/CCL18, Apo A-1 , transthyretin, CFH, TNF-a, IP-10, antithrombin III, GDF-15 and HCC1.

In one embodiment, the other biomarkers are selected from ferritin, SAP, A2M, IL-22, CFH, ECM-1, IL-1 β, sCD40L, IL-13, Apo-A-1 , myoglobin, IL-12(p40), MIP-4/CCL18, antithrombin III, GDF-15 and HCC1.

In one embodiment, one of the other biomarkers is ferritin. Alternatively, or in addition, one of the other biomarkers is SAP, one of the other biomarkers is A2M, and/or one of the other biomarkers is IL-22.

Examples of suitable panels of biomarkers which can be used to diagnose TB in the method of the present invention include:
NCAM, SAP, ferritin, CFH and ECM-1;
NCAM, SAP, IL-1 β, sCD40L, IL-13 and Apo A-1;
NCAM, A2M, IL-22, ferritin, myoglobin and IL-12(p40);
NCAM, A2M, IL-22, ferritin, TNF-β and MIP-4/CCL18;
NCAM, SAP, ferritin, PCT, MIG, TPA, GDF-15, HCC1, MIP-4/CCL18, antithrombin III;
NCAM, CRP, IFN-γ, IP-10, Apo A-1, SAA and CFH;
NCAM, CRP, IFN-Y, IP-10, Apo A-1 and CFH; and
NCAM, CRP, IFN-Y, IP-10, Apo A-1, CFH and ferritin.

The subject may be diagnosed as having TB if a reduced level of NCAM is detected in the sample relative to a level of NCAM which is associated with subjects with upper and lower respiratory tract infections, chronic obstructive pulmonary disease and asthma, and if at least one of the additional biomarkers is detected in the sample, or if at least two, at least three, at least four, at least five or at least six of the additional biomarkers are detected in the sample.

The sample is typically a blood sample containing immune cells, and can be a whole blood sample or a plasma or serum sample. The sample does not need to be stimulated with any antigens before it is tested.

The TB can be pulmonary TB or extra-pulmonary TB.

Capture agents specific for each of the biomarkers can be used to bind each of the respective biomarkers. One or more indicators can be provided to indicate when binding of each of the capture agents and biomarkers occurs.

Whole blood, serum and plasma are usually readily available from subjects and therefore the method of the present invention is suited to a point-of-care test based on the detection of biomarkers in these samples.

A capture agent and indicator can be used to bind to each of the biomarkers, and an indicator indicates when binding of the capture agents and each of the biomarkers occurs.

The biomarkers can be detected using commercially available techniques, such as ELISA techniques or multiplex bead array technology, although it is intended that a specific point-of-care diagnostic device will be developed for performing the method, particularly for use in resource poor settings.

Cut-off or threshold values can be determined based on levels of biomarkers which are typically found in patients without TB, and the levels of the biomarkers detected in the sample can be compared to the cut-off levels when making the determination of whether or not the subject has TB. In other words, the method will detect whether the biomarkers in the panels are under- or over-expressed relative to a subject who does not have TB.

Antibodies, affybodies, ankyrin repeat proteins, armadillo repeat proteins, nucleic acid aptamers, modified nucleic acid aptamers, peptides, modified peptides, carbohydrate ligands, synthetic ligands or synthetic polymers can be used as the capture agents, and the indicator can be a calorimetric, electrochemical, chromogenic, optical, fluorescent or a radio-labeled indicator.

The method can be used as an initial diagnostic tool whereby a positive diagnosis from this method can, if necessary, be subsequently confirmed by means of a second diagnostic method. In the interim, while waiting for the results of the second test, the subject can be started on treatment. Conversely, the method of the invention can also be used to rule out TB, thus preventing overtreatment of non-TB subjects.

The method of the invention can be performed using a diagnostic device which detects and indicates the presence of the biomarkers in the sample. The device has a means for receiving the sample from the subject, such as a loading or receiving area onto or into which the sample is placed. The capture agents and indicators are present in the device, and once the sample has been loaded onto or received into the device, the sample is brought into contact with the capture agents, which are allowed to bind to the biomarkers if present. The indicator will signify that binding has occurred. The device is typically a point-of-care device, such as a lateral flow device.

The device may be a dip-stick which can be dipped into the plasma or serum sample or onto which the sample can be placed, similar to many home pregnancy test kits. The capture agents are included on the dipstick, and generate a signal when they bind to the biomarkers in the panel, together with a control signal.

One example of a suitable point-of-care screening test is based on user-friendly lateral flow technology as recently demonstrated in a multi-centered African study [44]. While the sample can be plasma or serum, a whole blood sample (such as from a finger-prick) will be preferable. A nurse or front-line/first contact community healthcare worker can collect finger-prick blood from a patient suspected of having TB disease based on the symptoms, dilute the sample in a buffer which is provided in a kit, and add this to a membrane/test strip containing multiple biomarkers (or antibodies thereto) that have been coupled to up-converting phosphor (UCP) reporter particles. The blood can be left to flow on the strip for a few minutes and after it is dry, can be scanned using a portable hand-held device, typically one which is battery-operated (such as the UCP-Quant strip scanner reader, adapted from the ESE Quant LFR strip reader, Qiagen Lake Constance GmbH).

UCP reporter technology utilizes nano-sized or sub-micron particles that are composed of rare earth lanthanides in a crystal. The particles display a distinct anti-stokes behaviour upon excitation with infrared (IR) light: low-energy IR light is upconverted to higher energy visible light, with the emission spectrum dependent on the embedded rare earth components. The particles have an unmatched contrast as any possible autofluorescence is absent.

The assay format can apply sandwich-based immunochromatography with a complementary pair of chemokine-specific antibodies. Utilisation of dry UCP reporter material and lyophilized assay buffer allows convenient storage and transport of assay materials at ambient temperature.

Use of a kit for diagnosing tuberculosis can be provided to enable health workers to easily perform the diagnosis in a field setting. The kit can include a means for obtaining the blood sample from the patient (such as a needle, syringe or finger-prick device), optionally a blood sample collection device (such as a tube or capillary tube), a buffer for mixing with the blood sample, and a membrane/test strip with antibodies for the biomarker(s).

The invention will now be described in more detail by way of the following non-limiting examples.

### Examples:

### Materials and methods

### Study participants

Participants enrolled into the present study were individuals who presented with signs and symptoms requiring investigation for TB disease at the Fisantekraal Community Clinic in the outskirts of Cape Town, South Africa. The study was a sub-study of a larger diagnostic biomarker project (the African European Tuberculosis Consortium), that was ongoing at the study site and at field sites situated in six other African countries (www.ae-tbc.eu). All study participants presented with persistent cough lasting≥2 weeks and at least one of either fever, malaise, recent weight loss, night sweats, knowledge of close contact with a TB patient, haemoptysis, chest pain or loss of appetite. Participants were eligible for the study if they were 18 years or older and willing to give written informed consent for participation in the study, including consent for HIV testing. Patients were excluded if they were pregnant, had not been residing in the study community for more than 3 months, were severely anaemic (haemoglobin <10 g/I), were on anti-TB treatment, had received anti-TB treatment in the previous 90 days or if they were on quinolone or aminoglycoside antibiotics during the past 60 days. The study was approved by the Health Research Ethics Committee of the Faculty of Medicine and Health Sciences of the University of Stellenbosch.

### Sample collection and diagnostic tests

At enrolment, 6ml of blood was collected into heparinized BD vacutainer tubes (BD Biosciences) and transported to the laboratory at 4-8°C for further processing. Upon receipt in the laboratory, tubes were centrifuged at 2000 rpm for 10 minutes after which plasma was harvested, aliquoted and stored at -80 °C until analysed. Sputum samples were collected from all study participants and cultured using the MGIT method (BD Biosciences). Positive MGIT cultures were examined for acid fast bacilli using the Ziehl-Neelsen technique (to check for contamination), followed by Capilia TB testing (TAUNS, Numazu, Japan), to confirm the isolation of organisms of the M.tb complex, before being designated as positive cultures.

### Classification of study participants and reference standard

Participants were classified as definite TB cases, probable TB cases, participants with other respiratory diseases (ORD) or questionable disease status using a combination of clinical, radiological, and laboratory findings [8]. Only definite TB cases (culture positive individuals) and those with ORD were included in the study. Individuals with ORD had a range of other diagnoses, including upper and lower respiratory tract infections (viral and bacterial infections, although attempts to identify organisms by bacterial or viral cultures were not made), and acute exacerbations of chronic obstructive pulmonary disease or asthma. Because of the disproportionately high number of individuals with ORD, all 22 culture positive TB cases that were available at the study site were included in the study, as were 33 randomly selected individuals with ORD from the study biobank. In total, 55 study participants (22 of whom were culture positive TB cases) were investigated. The mean age of all study participants was 35.8 ± 10.2 years and 14 (25%) were HIV infected. The clinical and demographic characteristics of study participants are shown in Table 1.

**Table 1: Clinical and demographic characteristics of study participants**

| **Number of participants** | **All (n=55)** | **TB (n=22)** | **ORD (n=33)** |
|---|---|---|---|
| Males, n (%) | 22 (40) | 7 (32) | 15 (45) |
| Mean age, (Years)±SD | 35.8 ± 10.2 | 38.8 ±10.1 | 33.9 ± 9.9 |
| HIV Infected, n (%) | 14(25) | 4(18) | 10(30) |
| Quantiferon results | | | |
| Positive, n (%) | 34 (64) | 15(75) | 19 (58) |
| Negative, n (%) | 18 (34) | 4 (20) | 14 (42) |
| Indeterminate, n (%) | 1 (2) | 1 (5) | 0 (0) |

| | | | |
|---|---|---|---|
| Abbreviations: TB= pulmonary tuberculosis, SD=standard deviation | | | |

### Luminex Multiplex Immunoassay

The concentrations of 74 host markers including alpha-2-macroglobulin (A2M) , haptoglobin, C-reactive protein (CRP), serum amyloid P (SAP), procalcitonin (PCT), ferritin, tissue plasminogen activator (TPA), fibrinogen, serum amyloid A (SAA) (kits purchased from Bio-Rad Laboratories, Hercules, CA, USA), vitronectin, extracellular matrix protein 1 (ECM1 ), antithrombin III, vitamin D binding protein (VDBP), sFas, granzyme A, sFasL, sCD137, granzyme B, perforin, myoglobin, ADAMTS13, P-selectin, lipocalin-2, growth differentiation factor (GDF) -15 , thrombopoietin (TPO), stem cell factor (SCF), B-cell attracting chemokine (BCA)-1 , epithelial neutrophil activating protein (ENA-78), thymic stromal lymphopoietin (TSLP), I-309(CCL-1 ), stromal cell derived factor- 1 alpha (SDF-1a), interferon (IFN)-y, IFN-a2, interferon gamma inducible protein (IP)-10 (CXCL10), macrophage inflammatory protein (MIP)-1 β, tumor necrosis factor (TNF)-a, TNF-β, vascular endothelial growth factor (VEGF), soluble CD40 ligand (sCD40L), apolipoprotein (Apo) A-1 , Apo CIII, complement component 3 (CC3), transthyretin, complement factor H (CFH), total plasminogen activator inhibitor-1 (PAI-1 ), neural cell adhesion molecule (NCAM), brain-derived neurotrophic factor (BDNF), cathepsin D, myeloperoxidase (MPO), matrix metalloproteinase (MMP)-2, MMP-9, monokine induced by gamma interferon (MIG/CXCL9), granulocyte chemotactic protein-2 (GCP2), interferon inducible T-cell alpha chemoattractant (I-TAC/CXCL1 1 ), hemofiltrate CC chemokine-1 (HCC1 ), a1 -antitrypsin, pigment epithelium derived factor (PEDF), macrophage inflammatory protein-4 (MIP-4/CCL18), complement C4, interleukin (IL)-17F, IL-17A, IL-22, IL-33, IL-21 , IL-23, IL-25, IL-31 , IL-28A, IL-16, IL-I β, IL-12(p40), IL-13, IL-1 1 and IL-29 (Merck Millipore, Billerica, MA, USA), were investigated in plasma samples from all the study participants. The experiments were performed blindly, according to the instructions of the kit manufacturers, on the Bio-Plex platform (Bio-Rad). The Bio-Plex manager Software version 6.1 was used for bead acquisition and analysis of median fluorescence intensities.

### Statistical analysis

Differences in the concentrations of host markers between TB patients and individuals with ORD were analysed using the Mann-Whitney U test. The diagnostic abilities of individual host markers were assessed by receiver operator characteristics (ROC) curve analysis. The predictive abilities of combinations of host markers were investigated by general discriminant analysis (GDA), with leave-one-out cross validation [13]. Differences in the expression profiles of host markers during the course of TB treatment were analysed using mixed model repeated measures analysis of variance (ANOVA), with Fisher's Least Significant Difference (LSD) post hoc testing. P-values ≤0.05 were considered significant. The data were analysed using Statistica (Statsoft, Ohio, USA) and Graphpad Prism version 5 (Graphpad Software Inc., CA, USA).

### Results

### Utility of individual host markers in the diagnosis of TB disease

When the baseline concentrations of host markers in TB patients (n=22) were compared to the levels detected in patients with ORD (n=33), by the Mann Whitney U test, the concentrations of 23 out of the 74 analytes were significantly different between the two groups. The median levels of CRP, SAP, PCT, ferritin, TPA, SAA, ADAMTS-13, p-selectin, GDF-15, I-309, IFN-Y, IP-10, TNF-a, CFH, MIG, ITAC, HCC-1 and MIP-4 were significantly higher in TB cases, whereas the levels of antithrombin III, Apo A-1 , transthyretin, NCAM and BDNF were significantly higher in the ORD group. Trends (0.05<p≤ 0.01) towards higher levels of sFas, lipocalin-2, VEGF, PEDF, CC4 and IL-33 were observed in TB cases (Table 2). When the diagnostic accuracies of individual host markers were investigated by ROC curve analysis, the area under the ROC curve (AUC) was≥0.70 for 18 markers (Table 2). The most accurate single host markers included CRP, SAP, NCAM, TPA, I-309, and MIG, which all performed with AUC≥ 0.80 (Table 2, Figures 1 and 2). When data was stratified according to HIV infection status, concentrations of three additional markers (A2M, MIP-1 β and VEGF) became significant in the two groups, with AUC's of 0.70, 0.69 and 0.69 respectively.

### Utility of multi-plasma marker biosignatures in the diagnosis of TB disease

When the data obtained from all the TB patients and those with ORD were fitted into General Discriminant Analysis (GDA) models regardless of HIV status, combinations of up to six different host markers showed potential in the diagnosis of TB disease. A five-marker biosignature of NCAM, SAP, ferritin, CFH and ECM-1 diagnosed TB disease with a sensitivity of 95.2% (95% Cl, 81 .0-99.9%) and specificity of 92.9% (95% Cl, 70.8-98.9%) in the resubstitution classification matrix and sensitivity of 95.2% (95% Cl, 81 .0-99.9%) and specificity of 89.3% (95% Cl, 66.4-97.2%) after leave-one-out cross validation. However, the most optimal diagnostic biosignature (irrespective of HIV status) was a combination between six markers (NCAM, SAP, IL-1 β, sCD40L, IL-13 and Apo A-1 ), which diagnosed TB disease with a sensitivity 100% (95% Cl, 86.3-100%) and specificity of 89.3% (95%CI, 67.6-97.3%) after leave-one-out cross validation. The positive and negative predictive values of the six- marker biosignature were 87.5% (95% Cl, 66.5-96.7%) and 100% (95%CI, 83.4-100%) respectively (Figure 3).

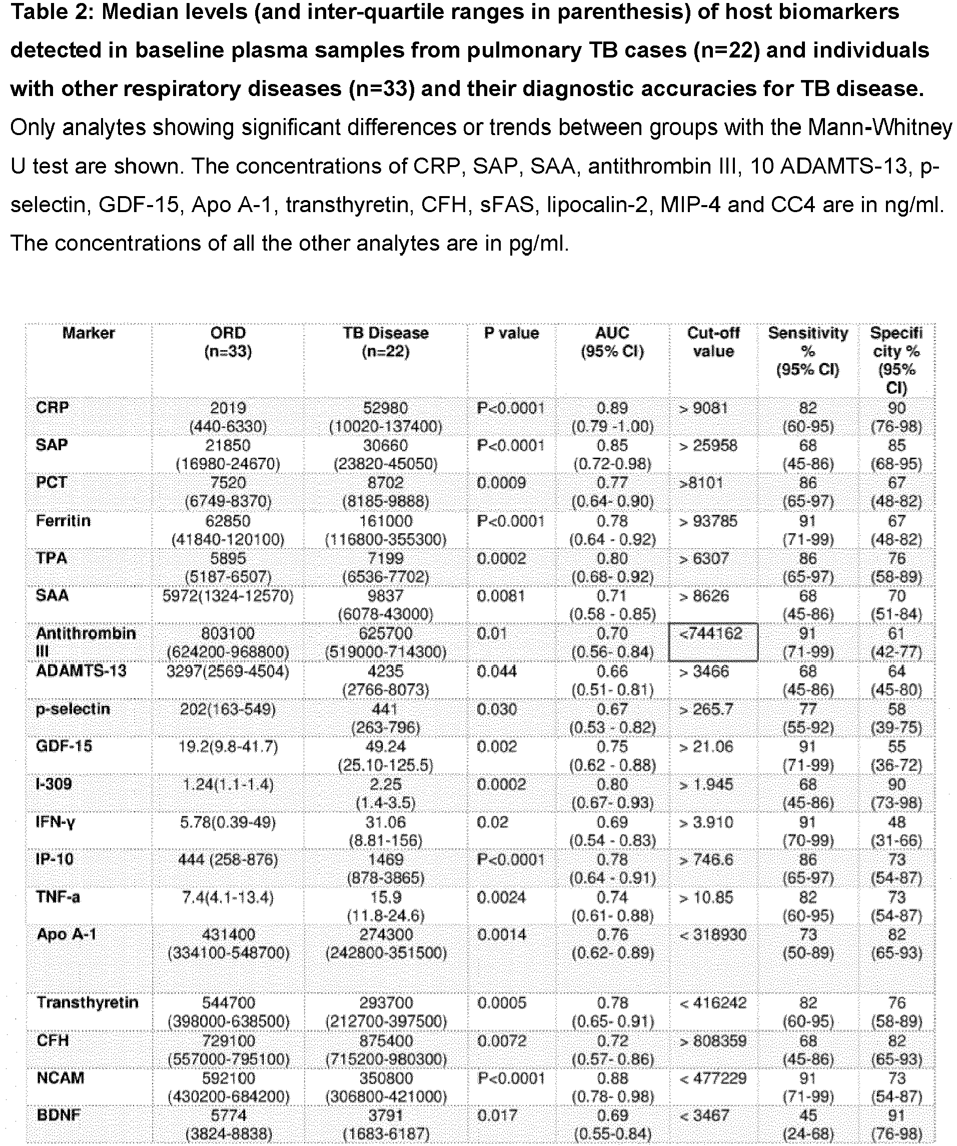

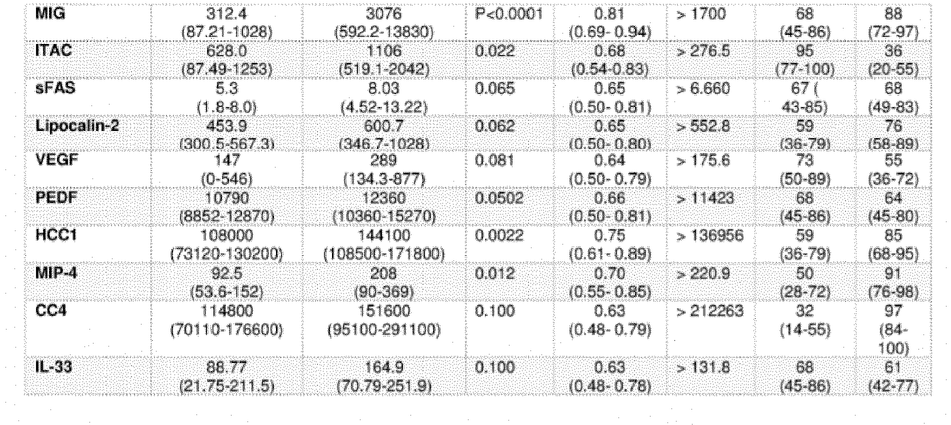

When the GDA procedure was repeated after excluding the HIV infected individuals, two six-marker biosignatures:- NCAM, A2M, IL-22, ferritin, myoglobin and IL-12(p40), and NCAM, 5 A2M, IL-22, ferritin, TNF-β and MIP-4, diagnosed TB disease with both sensitivity and specificity of 100% (AUC = 1 .0, 95% Cl, 1 .0-1 .0). NCAM was the most frequent analyte in biosignatures, appearing in all the top 13 biosignatures for diagnosing TB disease regardless of HIV infection status, and in 68%(23 of the 34) biosignatures that were generated for the diagnosis of TB disease after excluding HIV infected individuals. Other markers that occurred 10 most frequently in diagnostic biosignatures for TB disease included GDF-15, SAP, CFH, A2M, TNF-β, ferritin, SDF-1 a amongst others (Figure 3).

### Changes in host biomarker levels during the course of TB treatment (not part of the present invention)

To investigate whether any of the 74 markers could potentially be used to monitor the 15 response to TB treatment, the host markers were evaluated in plasma samples that were collected from TB patients at the end of standard TB treatment (month 6). However, of the 22 TB patients that were investigated in this study, only 15 (68%) returned to the clinic and provided samples at the end of treatment. Compared to baseline levels, the concentrations of 1 1 host markers changed significantly during the course of treatment. There was a significant 20 decrease in the levels of CRP, SAP, ferritin, IFN-γ, VEGF, IP-10, CC3, CFH and a-1 - antitrypsin from baseline to month 6, whereas a significant increase in the levels of transthyretin and MMP-2 was observed. The levels of IL-I β, SAA, sFas and MIG showed trends towards decreasing levels from baseline to month 6, whereas Apo-CIII, Apo A-1 and GCP-2 showed trends towards increasing levels at the end of treatment (Figures 4 to 12).

### Discussion

The diagnostic potentials of 74 host markers in plasma samples that were obtained from confirmed active TB cases and individuals with ORD were investigated as candidates for the diagnosis of TB disease. 18 of the 74 host markers (including new biomarkers in the TB field antithrombin III, GDF-15, NCAM, HCC1 and recently identified markers MIP-4, I-309, MIG, Apo A-1, transthyretin and CFH) showed potential in the diagnosis of TB disease, regardless of HIV infection status, as determined by area under the ROC curve (AUC). The most optimal diagnostic biosignature (irrespective of HIV infection status) was a six-marker model of NCAM, SAP, IL-1 β, sCD40L, IL-13 and Apo A-1 , which diagnosed TB disease with a sensitivity of 100% and specificity of 89.3%, with promising positive and negative predictive values. In the absence of HIV infection, six-marker biosignatures diagnosed TB disease with 100% accuracy.

Amongst the 18 host markers that showed the most potential in the diagnosis of TB disease individually as determined by AUC, six (CRP, SAP, PCT, ferritin, TPA and SAA) were acute phase proteins, six (I-309, MIG, MIP-4, Apo A-1 , transthyretin and CFH) were markers that play various roles in the body and have recently been identified as promising TB diagnostic candidates [14][10], two (TNF-a and IP-10) are widely investigated TB biomarkers [15], and four (antithrombin III, GDF-15, NCAM, HCC1 ) are markers which have not previously been investigated in the TB field. Other host markers (including ECM-1, IL-1 β, sCD40L and IL-13), although not very promising individually in the current study, were included into diagnostic biosignatures for TB disease.

Acute phase proteins are primarily produced by the liver, and act as opsonins at inflammatory sites [16]. Serum CRP has been extensively studied and shown to be promising for both the diagnosis of TB disease and monitoring of TB treatment response[17][18]. SAP, a homologue of CRP, has also been shown to have a protective role against bacterial infections [19]. Ferritin is a well-recognised protein in iron storage processes. It has long been established that iron acquisition is an essential virulence mechanism for pathogenic bacteria [20][21]. The concentrations of all the acute phase proteins that showed potential in the current study were elevated in the TB patients, and this is in agreement with previous findings [10][21 ][22].

The chemokines; MIG (CXCL9), IP-10(CXCL10), ITAC(CXCL11) and I-309(CCL-1) are found in abundance in activated bronchial epithelium[23]. MIP-4(CCL18) is a chemokine that is produced mainly by antigen presenting cells [24]. These chemokines play vital roles in the recruitment of activated T-cells to the site of infection [23][25]. The present study found significantly higher levels of these chemokines in the plasma of patients with active TB. The relatively new host markers investigated in this study (GDF-15, antithrombin III, HCC1 and NCAM) showed individual potential in the diagnosis of TB disease. Other markers (including p-selectin, ADAMTS-13 and BDNF), although not amongst the most promising single markers as demonstrated by ROC curve analysis, were significantly different between the TB patients and individuals with ORD, with the Mann-Whitney U test. GDF-15 is a member of the transforming growth factor beta superfamily (TGF-β) and its expression is associated with tissue damage, but has also been reported to exhibit tissue protective functions [28][29]. It has been identified as a prognostic marker for prostate cancer, with high serum levels observed in patients with liver cirrhosis and hepatocellular carcinoma [30][31]. Antithrombin complexes are important mediators of the coagulation system, with antithrombin III being one of the most important inhibitors of this system [32]. Markedly lowered antithrombin III plasma levels have been observed in sepsis [33]. HCC1 has been identified as a monocyte chemoattractant, with high concentrations observed in patients with chronic renal failure [34]. P-selectin is part of the selectin family of cell adhesion molecules, that promotes inflammatory reactions [35]. ADAMTS-13 is a metalloprotease with thrombospondin repeats, and has shown low activity in patients with recurrent thrombaotic thrombocytopenic purpura [36][37]. In a previous study by Liu et al, GDF-15 levels were not significantly different between TB patients, latently infected individuals and healthy controls [31]. However, GDF-15 levels showed potential in the current study. The current study observed significantly higher levels of p-selectin in TB patients in comparison to individuals with ORD. For the other newly identified TB diagnostic candidates, higher levels of HCC1 and ADAMTS-13 were observed in TB patients, but the levels of antithrombin III and NCAM were higher in individuals with ORD.

NCAM (CD56) is important in cell-cell or cell-matrix interactions, and is involved in neuronal differentiation, branching and survival [39]. It has been shown to play a role in lung tumor progression [40]. In the present study, the potential of NCAM as a biomarker for TB disease was demonstrated for the first time. NCAM was the most frequently occurring marker in biosignatures for the diagnosis of TB disease, and was included in all top 13 marker combinations for the diagnosis of TB disease, regardless of HIV infection status, and in 68% of the models that were generated when HIV infected individuals were excluded. The combination of NCAM with five other markers (SAP, IL-1 β, sCD40L, IL-13 and Apo A-1) diagnosed TB disease with high accuracy, regardless of HIV infection status, and all the TB patients and individuals with ORD were accurately classified (100% sensitivity and specificity) when NCAM was used in combination with either A2M+IL-22+ferritin+myoglobin+IL-12(p40), or A2M+IL-22+ferritin+TNF^+MIP-4, in the absence of HIV infection. In a previous serum-based study by the applicant [10], optimal diagnosis of TB disease was achieved using a
biosignature of CRP, IFN-γ, IP-10, Apo A-1 , SAA and CFH on a serum sample. There was excellent agreement between the findings of this and the serum study, as the diagnostic potential observed for individual host markers including CRP, SAA, SAP, CFH, Apo A-1, and ferritin amongst others was replicated in the current study. It is therefore envisaged that the new host markers identified in this study could be used in conjunction with, or as alternatives to, the markers that were included in the previous biosignature [10] if a point-of-care screening test based on these biosignatures were to be developed.

In addition to potentially being useful as diagnostic candidates for TB disease, 1 1 of the markers (including CRP, SAP, ferritin, IP-10, a-1 -antitrypsin) changed with treatment, thereby indicating that they may be potential candidates for monitoring the response to TB treatment. Although observations for MMP-2 (increasing levels from baseline to month 6) were contrary to the observations of Ugarte-Gil et al (decreasing levels from baseline to month 6 in sputum culture positive individuals after 2 weeks of treatment)[41], the observations for CRP, SAP, ferritin, IP-10, and a-1 -antitrypsin are in agreement with findings from previous studies[42][43].

### References:

1. WHO.Global Tuberculosis Report, 2015.
2. Desikan P: Sputum smear microscopy in tuberculosis: Is it still relevant? Indian J Med Res 2013, 137:442-444.
3. Home DJ, Royce SE, Gooze L, Narita M, Hopewell PC, Nahid P, Steingart KR: Sputum monitoring during tuberculosis treatment for predicting outcome: systematic review and metaanalysis. Lancet Infect D/'s 2010, 10:387-394.
4. Zeka AN, Tasbakan S, Cavusoglu C: Evaluation of the GeneXpert MTB/RIF Assay for Rapid Diagnosis of Tuberculosis and Detection of Rifampin Resistance in Pulmonary and Extrapulmonary Specimens. J Clin Microbiol 201 1, 49:4138-4141 .
5. Miotto P, Bigoni S, Migliori GB, Matteelli A, Cirillo DM: Early tuberculosis treatment monitoring by Xpert(R) MTB/RIF. Eur Respir J 2012, 39:1269-1271.
6. Denkinger CM, Pai M, Patel M, Menzies D: Gamma Interferon Release Assay for Monitoring of Treatment Response for Active Tuberculosis: an Explosion in the Spaghetti Factory. J Clin Microbiol 2013, 51:607-61 0.
7. Higuchi K, Harada N, Mori T: Interferon-y responses after isoniazid chemotherapy for latent tuberculosis. Respirology 2008, 13:468-472.
8. Jacobs R, Tshehla E, Malherbe S, Kriel M, Loxton AG, Stanley K, van der Spuy G, Walzl G, Chegou NN: Host biomarkers detected in saliva show promise as markers for the diagnosis of pulmonary tuberculosis disease and monitoring of the response to tuberculosis treatment. Cytokine 201 6, 81:50-56.
9. Phalane KG, Kriel M, Loxton AG, Menezes A, Stanley K, van der Spuy GD, Walzl G, Chegou NN: Differential Expression of Host Biomarkers in Saliva and Serum Samples from Individuals with Suspected Pulmonary Tuberculosis. Mediators Inflamm 2013, 2013:1 -1 0.
10. Chegou, N, Walzl. G, Mihret.A: Method for diagnosing Tuberculosis. International Patent Application no: PCT/IB2015/051435. https://patentscope.wipo. int/search/en/detail.jsf?docld=WO2015128830. [Accessed 1 April 2016]
11. Walzl G, Ronacher K, Hanekom W, Scriba TJ, Zumla A: Immunological biomarkers of tuberculosis. Nat Rev Immunol 201 1, 11 :343-354.
12. De Groote MA, Nahid P, Jarlsberg L, Johnson JL, Weiner M, Muzanyi G, Janjic N, Sterling DG, Ochsner UA: Elucidating Novel Serum Biomarkers Associated with Pulmonary Tuberculosis Treatment. PLoS ONE 2013, 8:e61 002.
13. General Discriminant Analysis - Statistics Textbook [http://documents.software.dell.com/Statistics/Textbook/General-Discriminant-AnalysisllAccessed 1 April 2016]
14. Chen T, Li H, Lin J, Guo H, Wang W, Chen L, Zhang X, Wang Y, Chen Y, Liao Q, others: Profiling the Human Immune Response to Mycobacterium Tuberculosis by Humane Cytokine Array. Tuberculosis 2016.
15. Chegou NN, Heyckendorf J, Walzl G, Lange C, Ruhwald M: Beyond the IFN-γ horizon: Biomarkers for immunodiagnosis of infection with M. tuberculosis. Eur Respir J 2013.
16. Gruys E, Toussaint MJM, Niewold TA, Koopmans SJ: Acute phase reaction and acute phase proteins. J Zhejiang Univ Sci 2005, 6B:1045-1056.
17. Shaikh MK, Samo JA, Devrajani BR, Shah SZA, Shaikh S, Shaikh I : C-reactive protein in patients with pulmonary tuberculosis. World Appl Sci J ' 2012, 17:140-144.
18. Rao S, Bernhardt V: Serum C-reactive protein in pulmonary tuberculosis: correlation with bacteriological load and extent of disease. Infect Dis Clin Pract 2009, 17:314-316.
19. Noursadeghi M, Bickerstaff MC, Gallimore JR, Herbert J, Cohen J, Pepys MB: Role of serum amyloid P component in bacterial infection: protection of the host or protection of the pathogen. Proc Natl Acad Sci 2000, 97:14584-14589.
20. Kotru M, Rusia U, Sikka M, Chaturvedi S, Jain AK: Evaluation of serum ferritin in screening for iron deficiency in tuberculosis. Ann Hematol 2004, 83:95-100.
21. Opolot JO, Theron AJ, Anderson R, Feldman C: Acute phase proteins and stress hormone responses in patients with newly diagnosed active pulmonary tuberculosis. Lung 2015, 193:13-18.
22. Radovic M, Pejcic T, Stankovic I, Ristic L, Rancic M, Ciric Z: Acute-Phase Inflammatory Response in Patients with Pulmonary Tuberculosis. Acta Fac Medicae Naissensis 2014, 31.
23. Sauty A, Dziejman M, Taha RA, larossi AS, Neote K, Garcia-Zepeda EA, Hamid Q, Luster AD: The T cell-specific CXC chemokines IP-10, Mig, and I-TAC are expressed by activated human bronchial epithelial cells. J Immunol 1999, 162:3549-3558.
24. Ferrara G, Bleck B, Richeldi L, Reibman J, Fabbri LM, Rom WN, Condos R: Mycobacterium tuberculosis Induces CCL18 Expression in Human Macrophages: CCL18 in Tuberculosis Infection. Scand J Immunol 2008, 68:668-674.
25. Haque NS, Fallon JT, Taubman MB, Harpel PC: The chemokine receptor CCR8 mediates human endothelial cell chemotaxis induced by I-309 and Kaposi sarcoma herpesvirus-encoded vMIP-I and by lipoprotein (a)-stimulated endothelial cell conditioned medium. Blood 2001 , 97:39-45.
26. Hong JY, Jung GS, Kim H, Kim YM, Lee HJ, Cho S-N, Kim SK, Chang J, Kang YA: Efficacy of inducible protein 10 as a biomarker for the diagnosis of tuberculosis. Int J Infect Dis 2012, 16:e855-e859.
27. Sauty A, Colvin RA, Wagner L, Rochat S, Spertini F, Luster AD: CXCR3 Internalization Following T Cell-Endothelial Cell Contact: Preferential Role of IFN-Inducible T Cell Chemoattractant (CXCL11). J Immunol 2001 , 167:7084-7093.
28. Hsiao EC, Koniaris LG, Zimmers-Koniaris T, Sebald SM, Huynh TV, Lee S-J: Characterization of growth-differentiation factor 15, a transforming growth factor β superfamily member induced following liver injury. Mol Cell Biol 2000, 20:3742-3751 .
29. Vanhara P, Hampl A, Kozubik A, Soucek K: Growth/differentiation factor-15: prostate cancer suppressor or promoter [quest]. Prostate Cancer Prostatic Dis 2012, 15:320-328.
30. Martin Boegemann FJW: GDF15 and Hepcidin as Prognostic Factors in Patients with Prostate Cancer. J Mol Biomark Diagn 2014, 05.
31. Liu X, Chi X, Gong Q, Gao L, Niu Y, Chi X, Cheng M, Si Y, Wang M, Zhong J, Niu J, Yang W: Association of Serum Level of Growth Differentiation Factor 15 with Liver Cirrhosis and Hepatocellular Carcinoma. PLOS OΛ/E 2015, 10:e0127518.
32. Levi M, Keller TT, van Gorp E, ten Cate H: Infection and inflammation and the coagulation system. Cardiovasc Res 2003, 60:26-39.
33. Mesters RM, Mannucci PM, Coppola R, Keller T, Ostermann H, Kienast J: Factor Vila and antithrombin III activity during severe sepsis and septic shock in neutropenic patients. Blood 1996, 88:881 -886.
34. Schulz-Knappe P, Magert HJ, Dewaid B, Meyer M, Cetin Y, Kubbies M, Tomeczkowski J, Kirchhoff K, Raida M, Adermann K, others: HCC-1 , a novel chemokine from human plasma. J Exp Med 1996, 183:295-299.
35. Wynn TA, Hesse M, Sandler NG, Kaviratne M, Hoffmann KF, Chiaramonte MG, Reiman R, Cheever AW, Sypek JP, Mentink-Kane MM: P-selectin suppresses hepatic inflammation and fibrosis in mice by regulating interferon ? and the IL-13 decoy receptor. Hepatology 2004, 39:676-687.
36. Soejima K, Mimura N, Hiroshima M, Maeda H, Hamamoto T, Nakagaki T, Nozaki C: A Novel Human Metalloprotease Synthesized in the Liver and Secreted into the Blood: Possibly, the von Willebrand Factor- Cleaving Protease? J Biochem (Tokyo) 2001 , 130:475-480.
37. Peyvandi F, Lavoretano S, Palla R, Feys HB, Vanhoorelbeke K, Battaglioli T, Valsecchi C, Canciani MT, Fabris F, Zver S, Reti M, Mikovic D, Karimi M, Giuffrida G, Laurenti L, Mannucci PM:ADAMTS13 and anti-ADAMTS13 antibodies as markers for recurrence of acquired thrombotic thrombocytopenic purpura during remission. Haematologica 2008, 93:232-239.
38. Mukae H, ASHITANI J, Tokojima M, Ihi T, Kohno S, Matsukura S: Elevated levels of circulating adhesion molecules in patients with active pulmonary tuberculosis. Respirology 2003, 8:326-331.
39. Kiryushko D, Korshunova I, Berezin V, Bock E: Neural cell adhesion molecule induces intracellular signaling via multiple mechanisms of Ca2+ homeostasis. Mol Biol Cell 2006, 17:2278-2286.
40. Campodonico PB, de Kier Joffe EDB, Urtreger AJ, Lauria LS, Lastiri JM, Puricelli LI, Todaro LB: The neural cell adhesion molecule is involved in the metastatic capacity in a murine model of lung cancer. Mol Carcinog 2010:n/a-n/a.
41. Ugarte-Gil CA, Elkington P, Gilman RH, Coronel J, Tezera LB, Bernabe-Ortiz A, Gotuzzo E, Friedland JS, Moore DAJ: Induced Sputum MMP-1 , -3 & -8 Concentrations during Treatment of Tuberculosis. PLoS ONE 2013, 8:e61333.
42. Martins C, Gama AC de C, Valcarenghi D, Batschauer AP de B: Markers of acute-phase response in the treatment of pulmonary tuberculosis. J Bras Patol E Med Lab 2014, 50.
43. Hong JY, Lee HJ, Kim SY, Chung KS, Kim EY, Jung JY, Park MS, Kim YS, Kim SK, Chang J, Cho S-N, Kang YA: Efficacy of IP-10 as a biomarker for monitoring tuberculosis treatment. J Infect 2014, 68:252-258.
44. Corstjens PLAM, Tjon Kon Fat EM, de Dood CJ, van der Ploeg-van Schip JJ, Franken KLMC, Chegou NN, Sutherland JS, Howe R, Mihret A, Kassa D, van der Vyver M, Sheehama J, Simukonda F, Mayanja-Kizza H, Ottenhoff THM, Walzl G, Geluk A: Multi-center evaluation of a user-friendly lateral flow assay to determine IP-10 and CCL4 levels in blood of TB and non-TB cases in Africa. Clin Biochem 2016, 49:22-31

## Claims

1. A method of diagnosing tuberculosis disease (TB) in a subject, the method comprising the steps of:
a) testing a blood sample from a subject suspected of having TB for the presence of neural cell adhesion molecule (NCAM/ CD56); and
b) diagnosing the patient as having TB if a reduced level of NCAM is detected in the sample relative to a level of NCAM which is associated with subjects with upper and lower respiratory tract infections, chronic obstructive pulmonary disease and asthma.

2. A method according to claim 1, which comprises the steps of:
a) in addition to testing said blood sample from a subject suspected of having TB for the presence of NCAM, testing a blood sample for at least one other biomarker selected from ferritin, serum amyloid P (SAP), alpha-2-macroglobulin (A2M), interleukin-22 (IL-22), complement factor H (CFH), extracellular matrix protein 1 (ECM-1), interleukin-1 β (IL-1 β), soluble CD40 ligand (sCD40L), interleukin-13 (IL-13), apolipoprotein A-1 (Apo-A-1), myoglobin, interleukin-12 (IL-12(p40)), macrophage inflammatory protein-4 (MIP-4/CCL18), antithrombin III, growth differentiation factor (GDF-15), hemofiltrate CC chemokine-1 (HCC1), C-reactive protein (CRP), procalcitonin (PCT), tissue plasminogen activator (TPA), serum amyloid A (SAA), a disintegrin and metalloproteinase with a thrombospondin type 1 motif, member 13 (ADAMTS-13 / von Willebrand factor-cleaving protease (VWFCP)), p-selectin, Chemokine (C-C motif) ligand 1 (CCL1 / I-309), interferon γ (IFN-γ), interferon gamma inducible protein 10 (IP-10), tumor necrosis factor α (TNF-α), monokine induced by gamma interferon (MIG), interferon inducible T-cell alpha chemoattractant (ITAC), transthyretin, brain-derived neurotrophic factor (BDNF), soluble Fas (sFas), lipocalin-2, vascular endothelial growth factor (VEGF), pigment epithelium derived factor (PEDF), complement component 4 (CC4), interleukin-33 (IL-33), tumor necrosis factor β (TNF-β), macrophage inflammatory protein 1β (MIP-1β) and stromal cell derived factor-1 alpha (SDF-1 α); and
b) diagnosing the patient as having TB if NCAM at a said reduced level and at least one of the biomarkers is detected in said sample.

3. A method according to claim 2, wherein the sample is tested for the presence of NCAM and:
at least two other biomarkers selected from ferritin, SAP, A2M, IL-22, CFH, ECM-1, IL-1β, sCD40L, IL-13, Apo-A-1, myoglobin, IL-12(p40), MIP-1β, CRP, PCT, TPA, SAA, ADAMTS-13, p-selectin, GDF-15, I-309, IFN-γ, IP-10, TNF-α, MIG, ITAC, HCC-1, antithrombin III, transthyretin, BDNF, sFas, lipocalin-2, VEGF, PEDF, CC4, IL-33, TNF-β and SDF-1 α;
at least three other biomarkers selected from ferritin, SAP, A2M, IL-22, CFH, ECM-1, IL-1 β, sCD40L, IL-13, Apo-A-1, myoglobin, IL-12(p40), MIP-4/CCL18, CRP, PCT, TPA, SAA, ADAMTS-13, p-selectin, GDF-15, I-309, IFN-γ, IP-10, TNF-α, MIG, ITAC, HCC-1, antithrombin III, transthyretin, BDNF, sFas, lipocalin-2, VEGF, PEDF, CC4, IL-33, TNF-β, MIP-1β and SDF-1 α;
at least four other biomarkers selected from ferritin, SAP, A2M, IL-22, CFH, ECM-1, IL-1 β, sCD40L, IL-13, Apo-A-1, myoglobin, IL-12(p40), MIP-4/CCL18, CRP, PCT, TPA, SAA, ADAMTS-13, p-selectin, GDF-15, I-309, IFN-γ, IP-10, TNF-α, MIG, ITAC, HCC-1, antithrombin III, transthyretin, BDNF, sFas, lipocalin-2, VEGF, PEDF, CC4, IL-33, TNF-β, MIP-1β and SDF-1 α; or
at least five other biomarkers selected from ferritin, SAP, A2M, IL-22, CFH, ECM-1, IL-1 β, sCD40L, IL-13, Apo-A-1, myoglobin, IL-12(p40), MIP-4/CCL18, CRP, PCT, TPA, SAA, ADAMTS-13, p-selectin, GDF-15, I-309, IFN-γ, IP-10, TNF-α, MIG, ITAC, HCC-1, antithrombin III, transthyretin, BDNF, sFas, lipocalin-2, VEGF, PEDF, CC4, IL-33, TNF-β, MIP-1β and SDF-1 α.

4. A method according to either of claims 2 or 3, wherein at least one of the other biomarkers is ferritin or SAP.

5. A method according to either of claims 3 or 4, wherein the sample is tested for the following biomarkers:
NCAM, SAP, ferritin, CFH and ECM-1;
NCAM, SAP, IL-1β, sCD40L, IL-13 and Apo A-1;
NCAM, A2M, IL-22, ferritin, myoglobin and IL-12(p40); or
NCAM, A2M, IL-22, ferritin, TNF-β and MIP-4/CCL18.

6. A method according to any one of claims 2 to 5, wherein the patient is diagnosed as having TB if at least two of the biomarkers are detected in the sample, if at least three of the biomarkers are detected in the sample, if at least four of the biomarkers are detected in the sample, if at least five of the biomarkers are detected in the sample, or if at least six of the biomarkers are detected in the sample.

7. A method according to any one of claims 1 to 6, wherein the sample is a plasma or serum sample.

8. A method according to any one of claims 1 to 7, wherein the sample is an unstimulated sample.

9. A method according to any one of claims 1 to 8, wherein the tuberculosis is pulmonary tuberculosis.

10. A method according to any one of claims 1 to 9, wherein capture agents specific for each of the biomarkers are used to bind each of the respective biomarkers and one or more indicators are provided to indicate when binding of each of the capture agents and biomarkers occurs.

11. Use of a kit for diagnosing tuberculosis in a blood sample from a subject according to the method of any one of claims 1 to 10, the kit comprising:
a) a means of receiving a sample from a patient;
b) a capture agent for binding NCAM and optionally also capture agents for binding other biomarkers selected from ferritin, SAP, A2M, IL-22, CFH, ECM-1, IL-1β, sCD40L, IL-13, Apo-A-1, myoglobin, IL-12(p40) and MIP-4/CCL18; and
c) at least one indicator which indicates when the capture agents bind to the biomarkers.

12. Use of a kit according to claim 11, wherein the kit includes:
a) means for obtaining the blood sample from the patient,
b) a blood sample collection device,
c) a buffer for mixing with the blood sample, and
d) a membrane/test strip with antibodies for the biomarker(s).

## Patentansprüche

1. Verfahren zur Diagnose von Tuberkuloseerkrankung (TB) bei einem Subjekt, wobei das Verfahren die folgenden Schritte umfasst:
a) Testen einer Blutprobe von einem Subjekt, bei dem das Vorliegen von TB vermutet wird, auf die Gegenwart von neuralem Zelladhäsionsmolekül (NCAM/ CD56); und
b) Diagnostizieren von TB bei dem Patienten, wenn ein reduzierter Spiegel von NCAM in der Probe in Bezug auf einen Spiegel von NCAM, der mit Subjekten mit Infektionen im oberen und unteren Respirationstrakt, chronisch obstruktiver Lungenerkrankung und Asthma verbunden wird, nachgewiesen wird.

2. Verfahren nach Anspruch 1, das die folgenden Schritte umfasst:
a) zusätzlich zum Testen der Blutprobe von einem Subjekt, bei dem das Vorliegen von TB vermutet wird, auf die Gegenwart von NCAM das Testen einer Blutprobe auf mindestens einen anderen Biomarker der aus Folgenden ausgewählt ist: Ferritin, Serum-Amyloid P (SAP), alpha-2-Makroglobulin (A2M), Interleukin-22 (IL-22), Komplementfaktor H (CFH), extrazellulärem Matrixprotein 1 (ECM-1), Interleukin-1β (IL-1β), löslichem CD40-Liganden (sCD40L), Interleukin-13 (IL-13), Apolipoprotein A-1 (Apo-A-1), Myoglobin, Interleukin-12 (IL-12(p40)), Makrophagen-Entzündungsprotein-4 (MIP-4/CCL18), Antithrombin III, Wachstums- und Differenzierungsfaktor (GDF-15), Hämofiltrat-CC-Chemokin-1 (HCC1), C-reaktivem Protein (CRP), Procalcitonin (PCT), Gewebeplasminogenaktivator (TPA), Serum-Amyloid A (SAA), einer Disintegrin- und Metalloproteinase mit einem Thrombospondin-Typ-1-Motiv, Mitglied 13 (ADAMTS-13/Von-Willebrand-Faktor-spaltender Protease (VWFCP)), P-Selektin, Chemokin (C-C-Motiv)-Ligand 1 (CCL1/I-309), Interferon-γ (IFN-y), Interferon-gamma-induzierbarem Protein 10 (IP-10), Tumornekrosefaktor α (TNF-α), gamma-Interferon-induziertem Monokin (MIG), Interferon-induzierbarem T-Zell-alpha-Chemoattraktant (ITAC), Transthyretin, vom Gehirn abgeleitetem neurotrophem Faktor (BDNF), löslichem Fas (sFas), Lipocalin-2, vaskulärem endothelialem Wachstumsfaktor (VEGF), Pigmentepithelium-abgeleitetem Faktor (PEDF), Komplementkomponente 4 (CC4), Interleukin-33 (IL-33), Tumornekrosefaktor β (TNF-ß), Makrophagen-Entzündungsprotein 1β (MIP-1(3) und Stromazell-abgeleitetem Faktor-1-alpha (SDF-1α), und
b) Diagnostizieren des Patienten mit TB, wenn NCAM bei dem reduzierten Spiegel und mindestens einer der Biomarker in der Probe nachgewiesen werden.

3. Verfahren nach Anspruch 2, wobei die Probe auf die Gegenwart von NCAM und Folgenden getestet wird:
mindestens zwei anderen Biomarkern, die aus Folgenden ausgewählt sind: Ferritin, SAP, A2M, IL-22, CFH, ECM-1, IL-1β, sCD40L, IL-13, Apo-A-1, Myoglobin, IL-12(p40), MIP-1β, CRP, PCT, TPA, SAA, ADAMTS-13, P-Selektin, GDF-15, 1-309, IFN-γ, IP-10, TNF-α, MIG, ITAC, HCC-1, Antithrombin III, Transthyretin, BDNF, sFas, Lipocalin-2, VEGF, PEDF, CC4, IL-33, TNF-β und SDF-1α;
mindestens drei anderen Biomarkern, die aus Folgenden ausgewählt sind: Ferritin, SAP, A2M, IL-22, CFH, ECM-1, IL-1β, sCD40L, IL-13, Apo-A-1, Myoglobin, IL-12(p40), MIP-4/CCL18, CRP, PCT, TPA, SAA, ADAMTS-13, P-Selektin, GDF-15, 1-309, IFN-γ, IP-10, TNF-α, MIG, ITAC, HCC-1, Antithrombin III, Transthyretin, BDNF, sFas, Lipocalin-2, VEGF, PEDF, CC4, IL-33, TNF-β, MIP-1β und SDF-1α;
mindestens vier anderen Biomarkern, die aus Folgenden ausgewählt sind: Ferritin, SAP, A2M, IL-22, CFH, ECM-1, IL-1β, sCD40L, IL-13, Apo-A-1, Myoglobin, IL-12(p40), MIP-4/CCL18, CRP, PCT, TPA, SAA, ADAMTS-13, P-Selektin, GDF-15, 1-309, IFN-γ, IP-10, TNF-α, MIG, ITAC, HCC-1, Antithrombin III, Transthyretin, BDNF, sFas, Lipocalin-2, VEGF, PEDF, CC4, IL-33, TNF-β, MIP-1β und SDF-1α; oder
mindestens fünf anderen Biomarkern, die aus Folgenden ausgewählt sind: Ferritin, SAP, A2M, IL-22, CFH, ECM-1, IL-1β, sCD40L, IL-13, Apo-A-1, Myoglobin, IL-12(p40), MIP-4/CCL18, CRP, PCT, TPA, SAA, ADAMTS-13, P-Selektin, GDF-15, 1-309, IFN-γ, IP-10, TNF-α, MIG, ITAC, HCC-1, Antithrombin III, Transthyretin, BDNF, sFas, Lipocalin-2, VEGF, PEDF, CC4, IL-33, TNF-β, MIP-1β und SDF-1α.

4. Verfahren nach einem der Ansprüche 2 oder 3, wobei mindestens einer der anderen Biomarker Ferritin oder SAP ist.

5. Verfahren nach einem der Ansprüche 3 oder 4, wobei die Probe auf die folgenden Biomarker getestet wird:
NCAM, SAP, Ferritin, CFH und ECM-1;
NCAM, SAP, IL-1β, sCH40L, IL-13 und Apo-A-1;
NCAM, A2M, IL-22, Ferritin, Myoglobin und IL-12(p40); oder
NCAM, A2M, IL-22, Ferritin, TNF-β und MIP-4/CCL18.

6. Verfahren nach einem der Ansprüche 2 bis 5, wobei der Patient mit TB diagnostiziert wird, wenn mindestens zwei der Biomarker in der Probe nachgewiesen werden, wenn mindestens drei der Biomarker in der Probe nachgewiesen werden, wenn mindestens vier der Biomarker in der Probe nachgewiesen werden, wenn mindestens fünf der Biomarker in der Probe nachgewiesen werden oder wenn mindestens sechs der Biomarker in der Probe nachgewiesen werden.

7. Verfahren nach einem der Ansprüche 1 bis 6, wobei die Probe eine Plasma- oder Serumprobe ist.

8. Verfahren nach einem der Ansprüche 1 bis 7, wobei die Probe eine nichtstimulierte Probe ist.

9. Verfahren nach einem der Ansprüche 1 bis 8, wobei die Tuberkulose Lungentuberkulose ist.

10. Verfahren nach einem der Ansprüche 1 bis 9, wobei für jeden der Biomarker spezifische Einfangmittel verwendet werden, um jeden der jeweiligen Biomarker zu binden, und ein oder mehrere Indikatoren bereitgestellt werden, um anzuzeigen, wenn eine Bindung von jedem der Einfangmittel und Biomarker auftritt.

11. Verwendung eines Kits zur Diagnose von Tuberkulose in einer Blutprobe von einem Subjekt gemäß dem Verfahren nach einem der Ansprüche 1 bis 10, wobei der Kit Folgendes umfasst:
a) ein Mittel zur Aufnahme einer Probe von einem Patienten,
b) ein Einfangmittel zur Bindung von NCAM und optional auch Einfangmittel zur Bindung anderer Biomarker, die aus Folgenden ausgewählt sind:
Ferritin, SAP, A2M, IL-22, CFH, ECM-1, IL-1β, sCD40L, IL-13, Apo-A-1, Myoglobin, IL-12(p40) und MIP-4/CCL18; und
c) mindestens einen Indikator, der anzeigt, wenn die Einfangmittel zu den Biomarkern binden.

12. Verwendung eines Kits nach Anspruch 11, wobei der Kit Folgendes einschließt:
a) Mittel zum Erhalten der Blutprobe von dem Patienten,
b) eine Blutprobensammelvorrichtung,
c) einen Puffer zum Mischen mit der Blutprobe und
d) eine Membran/einen Teststreifen mit Antikörpern für den/die Biomarker.

## Revendications

1. Procédé de diagnostic de la maladie tuberculose (TB) chez un sujet, le procédé comprenant les étapes consistant à :
a) tester un échantillon de sang d'un sujet soupçonné avoir la TB pour la présence de molécule d'adhésion cellulaire neuronale (NCAM/CD56) ; et
b) diagnostiquer le patient comme ayant la TB si un taux réduit de NCAM est détecté dans l'échantillon par rapport à un taux de NCAM qui est associé à des sujets ayant des infections des voies respiratoires supérieures et inférieures, une maladie pulmonaire obstructive chronique et l'asthme.

2. Procédé selon la revendication 1, qui comprend les étapes consistant à :
a) en plus de tester ledit échantillon de sang d'un sujet soupçonné avoir la TB pour la présence de NCAM, tester un échantillon de sang pour au moins un autre biomarqueur sélectionné parmi la ferritine, sérum amyloïde P (SAP), alpha-2-macroglobuline (A2M), interleukine 22 (IL-22), facteur H du complément (CFH), protéine 1 de la matrice extracellulaire (ECM-1), interleukine-1β (IL-1β, ligand CD40 soluble (sCD40L), interleukine-13 (IL-13), apolipoprotéine A-1 (Apo-A-1), myoglobine, interleukine-12 (IL-12(p40)), protéine inflammatoire des macrophages de type 4 (MIP-4/CCL18), antithrombine III, facteur de différenciation de croissance (GDF-15), chimiokine CC-1 d'hémofiltratration (HCC1), protéine C-réactive (CRP), procalcitonine (PCT), activateur tissulaire du plasminogène (TPA), sérum amyloïde A (SAA), une désintégrine et une métalloprotéinase avec un motif de thrombospondine de type 1, membre 13 (ADAMTS-13/protéase de clivage du facteur de Willebrand (VWFCP)), p-sélectine, ligand 1 (motif C-C) de chimiokine (CCL1/I-309), interféron γ (IFN-y), protéine 10 induite par l'interféron gamma (IP-10), facteur α de nécrose tumorale (TNF-α), monokine induite par l'interféron gamma (MIG), chimio-attracteur alpha des lymphocytes T induit par l'interféron (ITAC), transthyrétine, facteur neurotrophique dérivé du cerveau (BDNF), Fas soluble (sFas), lipocaline-2, facteur de croissance de l'endothélium vasculaire (VEGF), facteur dérivé de l'épithélium pigmentaire (PEDF), composant 4 du complément (CC4), interleukine-33 (IL-33), facteur β de nécrose tumorale (TNF-β), protéine inflammatoire des macrophages de type 1β (MIP-1β) et facteur 1 alpha dérivé des cellules stromales (SDF-1α) ; et
b) diagnostiquer le patient comme ayant la TB si la NCAM à un dit taux réduit et si au moins un biomarqueur est détecté dans ledit échantillon.

3. Procédé selon la revendication 2, dans lequel l'échantillon est testé pour la présence de NCAM et :
au moins deux autres biomarqueurs sélectionnés parmi la ferritine, SAP, A2M, IL-22, CFH, ECM-1, IL-Iβ, sCD40L, IL-13, Apo-A-1, myoglobine, IL-12(p40), MIP-1β, CRP, PCT, TPA, SAA, ADAMTS-13, p-sélectine, GDF-15, 1-309, IFN-γ, IP-10, TNF-α, MIG, ITAC, HCC-1, antithrombine III, transthyrétine, BDNF, sFas, lipocaline-2, VEGF, PEDF, CC4, IL-33, TNF-β et SDF-1α ;
au moins trois autres biomarqueurs sélectionnés parmi la ferritine, SAP, A2M, IL-22, CFH, ECM-1, IL-Iβ, sCD40L, IL-13, Apo-A-1, myoglobine, IL-12(p40), MIP-4/CCL18, CRP, PCT, TPA, SAA, ADAMTS-13, p-sélectine, GDF-15, 1-309, IFN-γ, IP-10, TNF-α, MIG, ITAC, HCC-1, antithrombine III, transthyrétine, BDNF, sFas, lipocaline-2, VEGF, PEDF, CC4, IL-33, TNF-β, MIP-1β et SDF-1α ;
au moins quatre autres biomarqueurs sélectionnés parmi la ferritine, SAP, A2M, IL-22, CFH, ECM-1, IL-Iβ, sCD40L, IL-13, Apo-A-1, myoglobine, IL-12(p40), MIP-4/CCL18, CRP, PCT, TPA, SAA, ADAMTS-13, p-sélectine, GDF-15, 1-309, IFN-γ, IP-10, TNF-α, MIG, ITAC, HCC-1, antithrombine III, transthyrétine, BDNF, sFas, lipocaline-2, VEGF, PEDF, CC4, IL-33, TNF-β, MIP-1β et SDF-1α ; ou
au moins cinq autres biomarqueurs sélectionnés parmi la ferritine, SAP, A2M, IL-22, CFH, ECM-1, IL-Iβ, sCD40L, IL-13, Apo-A-1, myoglobine, IL-12(p40), MIP-4/CCL18, CRP, PCT, TPA, SAA, ADAMTS-13, p-sélectine, GDF-15, 1-309, IFN-γ, IP-10, TNF-α, MIG, ITAC, HCC-1, antithrombine III, transthyrétine, BDNF, sFas, lipocaline-2, VEGF, PEDF, CC4, IL-33, TNF-β, MIP-1β et SDF-1α.

4. Procédé selon l'une ou l'autre des revendications 2 ou 3, dans lequel au moins l'un des autres biomarqueurs est la ferritine ou le SAP.

5. Procédé selon l'une ou l'autre des revendications 3 ou 4, dans lequel l'échantillon est testé pour les biomarqueurs suivants :
NCAM, SAP, ferritine, CFH et ECM-1 ;
NCAM, SAP, IL-1β, sCD40L, IL-13 et Apo-A-1 ;
NCAM, A2M, IL-22, ferritine, myoglobine et IL-12(p40) ; ou
NCAM, A2M, IL-22, ferritine, TNF-β et MIP-4/CCL18.

6. Procédé selon l'une quelconque des revendications 2 à 5, dans lequel le patient est diagnostiqué comme ayant la TB si au moins deux des biomarqueurs sont détectés dans l'échantillon, si au moins trois des biomarqueurs sont détectés dans l'échantillon, si au moins quatre des biomarqueurs sont détectés dans l'échantillon, si au moins cinq des biomarqueurs sont détectés dans l'échantillon ou si au moins six des biomarqueurs sont détectés dans l'échantillon.

7. Procédé selon l'une quelconque des revendications 1 à 6, dans lequel l'échantillon est un échantillon de plasma ou de sérum.

8. Procédé selon l'une quelconque des revendications 1 à 7, dans lequel l'échantillon est un échantillon non stimulé.

9. Procédé selon l'une quelconque des revendications 1 à 8, où la tuberculose est une tuberculose pulmonaire.

10. Procédé selon l'une quelconque des revendications 1 à 9, dans lequel des agents de capture spécifiques pour chacun des biomarqueurs sont utilisés pour se lier à chacun des biomarqueurs respectifs et un ou plusieurs indicateurs sont fournis pour indiquer quand la liaison de chacun des agents de capture et des biomarqueurs a lieu.

11. Utilisation d'un kit pour diagnostiquer la tuberculose dans un échantillon de sang d'un sujet conformément au procédé selon l'une quelconque des revendications 1 à 10, le kit comprenant :
a) un moyen pour recevoir un échantillon du patient ;
b) un agent de capture pour se lier à NCAM et optionnellement aussi des agents de capture pour se lier à d'autres biomarqueurs sélectionnés parmi la ferritine, SAP, A2M, IL-22, CFH, ECM-1, IL-Iβ, sCD40L, IL-13, Apo-A-1, myoglobine, IL-12(p40) et MIP-4/CCL 18 ; et
c) au moins un indicateur qui indique quand les agents de capture se lient aux biomarqueurs.

12. Utilisation d'un kit selon la revendication 11, où le kit comprend :
a) un moyen pour obtenir l'échantillon de sang du patient,
b) un dispositif de collecte d'échantillon de sang,
c) un tampon à mélanger avec l'échantillon de sang, et
d) une membrane/bande de test avec des anticorps pour le(s) biomarqueur(s).
